(19) Europäisches Patentamt
European Patent Office
Office européen des brevets

(11) **EP 3 054 844 B1**

(12) **EUROPEAN PATENT SPECIFICATION**

(45) Date of publication and mention
of the grant of the patent:
**29.07.2020 Bulletin 2020/31**

(51) Int Cl.:
**A61B 5/107** *(2006.01)*  **A61B 5/11** *(2006.01)*
**A61B 5/08** *(2006.01)*  **A61B 5/00** *(2006.01)*

(21) Application number: **14798972.7**

(22) Date of filing: **10.10.2014**

(86) International application number:
**PCT/IB2014/065197**

(87) International publication number:
**WO 2015/052683 (16.04.2015 Gazette 2015/15)**

(54) **DIFFICULT INTUBATION OR VENTILATION OR EXTUBATION PREDICTION SYSTEM**

VORHERSAGESYSTEM FÜR SCHWERE INTUBATION ODER BEATMUNG ODER EXTUBATION

SYSTÈME DE PRÉDICTION D'INCUBATION OU DE VENTILATION OU D'EXTUBATION DIFFICILE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.10.2013 EP 13188392**

(43) Date of publication of application:
**17.08.2016 Bulletin 2016/33**

(73) Proprietor: **Centre Hospitalier Universitaire Vaudois (CHUV)**
**1011 Lausanne (CH)**

(72) Inventors:
• **SCHOETTKER, Patrick**
**1005 Lausanne (CH)**
• **CUENDET, Gabriel**
**1700 Fribourg (CH)**
• **PERRUCHOUD, Christophe**
**1110 Morges (CH)**
• **SORCI, Matteo**
**1110 Morges (CH)**
• **THIRAN, Jean-Philippe**
**1614 Granges (Veveyse) (CH)**

(74) Representative: **reuteler & cie SA**
**Chemin de la Vuarpillière 29**
**1260 Nyon (CH)**

(56) References cited:
**US-A1- 2007 214 013    US-A1- 2010 312 144**

• **HONG-MEI YAN ET AL: "Predicting Cormack classification based on neural network with multiple anthropometric features", APPERCEIVING COMPUTING AND INTELLIGENCE ANALYSIS (ICACIA), 2010 INTERNATIONAL CONFERENCE ON, IEEE, 17 December 2010 (2010-12-17), pages 52-55, XP031901458, DOI: 10.1109/ICACIA.2010.5709849 ISBN: 978-1-4244-8025-8**

**Description**

**TECHNICAL FIELD**

The present invention relates to the field of automated systems for predicting the difficulty in intubating or ventilating an anesthetized patient, as well as predicting the post-operative airway complications, including sleep apnea syndrome

**DESCRIPTION OF RELATED ART**

After induction of general anaesthesia necessary to perform surgery, the patient necessitates facial mask ventilation followed by endotracheal intubation. Intubation comprises the placement of a flexible tube into the trachea to maintain open an airway and to allow artificial breathing. Facial mask ventilation is the application of a face mask on the patients' mouth and nose in order to administer supplemental oxygen. This face mask ventilation is necessary for 3-4 minutes to keep the patient alive (as he is not breathing spontaneously anymore) while the medications' effect allow intubation of the trachea.. Mechanical ventilation is then performed through the endotracheal tube with the help of a ventilator, to supply pressurised oxygen, air or anaesthetic gases to the patient.

The difficulty in intubating and / or ventilating varies between patients and remains a constant area of concern for anaesthetists, intensive care or emergency medicine physicians. For example, an upper airway may collapse after induction of general anaesthesia or a specific anatomical feature may make intubation difficult. In the same way face mask ventilation may be difficult due to anatomical patient factors, due to ineffective sealing around a face of a patient, or by excessive resistance to inlet / outlet of air.

After induction of general anaesthesia, the patient stops breathing and face mask ventilation followed by endotracheal intubation is mandatory. Multiple failed attempts to intubate / ventilate can lead to complications ranging from airway trauma, severe hypoxemic brain injuries to death of the patient. It is estimated that there are approximately 3 in 10'000 cases wherein a patient cannot be intubated and cannot be ventilated. In 2013, failure to intubate or to ventilate a patient still represents the main cause of morbidity and mortality in the practice of anaesthesia. After an operation and anesthesia is finished, it is proceeded to the extubation of the patient. Tracheal extubation is a high-risk phase of anaesthesia. Guidelines of management have been elaborated very recently, combining factors such as difficulty for intubation, airway status and operative conditions. Airway obstruction is the most common cause of airway-related events and need for reintubation in the early postoperative setting have been reported for rates up to 0.45%, puttzing the patients at serious risks.

Sleep apnea syndrome significantly increases the rate of post-operative failure and should be detected preoperatively.

Accordingly, patients are subject to a pre-operation airway examination looking for anatomical features that are potentially indicative of difficulty in ventilation or intubation or extubation. A range of examinations exist and typically at least two examinations are performed as part of the pre-operative examination. Two examples of dedicated examinations are discussed in the following.

One of the most common examinations is the Mallampati test, wherein an examination is performed of oropharyngeal structures that are visible when the seated patient fully opens their mouth and thereafter extends the tongue without phonation. The visibility of some oropharyngeal structures, such as the uvula is compared to other structures, such as the hard palate. The visibility is used to provide a grade from 1-4 with a theoretical correlation to the ease of intubation. In A. Lee, L. T. Y. Fan, T. Gin, M. K. Karmakar, and W. D. N. Kee, "A Systematic Review (Meta-Analysis) of the Accuracy of the Mallampati Tests to Predict the Difficult Airway," Anaesthesia & Analgesia, vol. 102, pp. 1867-1878, June 2006 [16], it was concluded that the Mallampati test's clinical value as a screening test was limited as it had poor to moderate discriminative power when used alone. Further details of the Mallampati test are provided in described in Mallampati, S. R. et al. Can. Anaesth. Soc. J. 1985; 32: 429-34 and Samsoon, G. L. et al. Anaesthesia 1987; 42: 487-90, which is incorporated herein by reference.

Another commonly used examinations is the thyromental distance test. The thyromental distance (TMD) is the distance from the upper edge of thyroid cartilage to the chin. The distance is measured with the head fully extended. A short thyromental distance theoretically equates with an anterior lying larynx that is at a more acute angle and potentially results in less space for the tongue to be compressed into by the laryngoscope blade necessary for the intubation. A thyromental distance greater than 7 cm is usually associated with easy intubation whereas a thyromental distance smaller than 6 cm may predict a difficult intubation. However, as discussed in P. A. Baker, A. Depuydt, and J. M. D. Thompson, "Thyromental distance measurement - fingers don't rule," Anaesthesia, vol. 64, pp. 878-882, Aug. 2009, with a sensitivity of 48% and a specificity of 79% in predicting difficult intubation, the thyromental distance is not a good predictor and is often used in conjunction with other predictors. Currently available screening tests for difficult intubation have only poor to moderate discriminative power when used alone. Combinations add some incremental diagnostic value. However, the clinical value of these bedside screening tests for predicting difficult intubation remains limited as written by Shiga et al. in the currently biggest meta-analysis on the subject of prediction of difficult intubation and published in Anesthesiology 2005 ;103 :429-437.

Difficult extubation has been associated with morphological factors, such as obesity and obstructive sleep apnea syndrome, head and neck pathology as well as pharyngeal and laryngeal obstruction.

US 8460215 discloses a system for predicting difficult intubation in a patient, wherein a camera is used to obtain a static image of a patient, the image is processed with face structural analysis software to determine a number of variables. The variables are then used by a predictive model to determine a difficult to intubate value, which may be within the range of 0-1. A drawback of such a system is that the system is limited to obtaining variables when a patient's face is static and it will be appreciated that some variables change as a patient moves.

The assessment of difficult intubation is therefore an important research topic in anaesthesia and it has been shown that some measures from the face and the neck can predict the difficulty with performances ranging from poor to relatively good.

In respect of mask ventilation, typically no predictive test of difficult ventilation is performed prior to anaesthesia. Difficult ventilation however also poses risks that would be advantageous to predict.

In respect of extubation difficulty, no robust predictive test of difficulty is performed, neither is sleep apnea syndrome detected routinely.

## SUMMARY OF THE INVENTION

An objective of the invention is to provide an accurate, reliable and safe predictive system for determining the difficulty in intubating and / or ventilation of a patient.

It would be advantageous to provide an accurate, reliable and safe predictive system for determining the difficulty in extubating a patient.

It would be advantageous to provide a predictive system that is convenient and easy to use.

It would be advantageous to provide a predictive system that is economical.

It would be advantageous to provide a predictive system that produces results rapidly. Objects of the invention are achieved by a predictive system according to claim 1. Objects of the invention are achieved by a predictive method according to claim 10.

The dependent claims describe various advantageous features of the invention.

Disclosed herein, according to a first aspect of the invention, is a system to determine the difficulty in intubating and / or ventilating a subject, the system comprising: an imaging module to acquire image parameters comprising a dynamic image of part of a head or neck of a subject; a parameter extraction module configured to extract at least one dynamic parameter from the image parameters; and a classification module comprising a classification algorithm configured to determine a difficulty to intubate and / or ventilate value based on the at least one dynamic parameter.

The system may also advantageously be configured to determine the difficulty in extubating a subject, whereby the classification algorithm is configured to determine a difficulty to extubate value based on the at least one dynamic parameter.

In an embodiment, the imaging module is configured to acquire image parameters comprising a head movement sequence about a neck of a subject and the dynamic parameter is determined from the head and / or neck movement.

The head movement about the neck may comprise one or more of the following movements: a head up and / or a head down movement; a head rotate to the left and / or a head rotate to the right movement; a head translational movement and / or a head arcing movement.

In an embodiment, the imaging module is configured to acquire image parameters comprising one or more of the following movements: a jaw movement; a tongue movement; lip bite movement.

The system may further comprise an imaging unit, the imaging unit being operable to capture a moving image of a subject.

In an embodiment, the parameter extraction module comprise an optic flow module for processing the dynamic parameters, the optic flow module to process the image parameters to map the direction and movement of the subject in 3D and / or 2D optic flow representations.

In an embodiment, the parameter extraction module comprise a filter module, the filter module to process the optic flow data to remove sources of noise.

In an embodiment, the parameter extraction module comprises a dynamic parameter determination module, the dynamic parameter determination module to determine one or more dynamic parameters from the optic flow data.

The movement being captured may be a head up and / or a head down movement and the dynamic parameter may comprise one or more of the following: extension angle; movement magnitude; motion coherence index.

In an embodiment, the imaging system is further operable to provide data comprising a static image of part of a head or neck of a user; the parameter extraction module being further configured to extract at least one static parameter from the static image parameters; the classification algorithm being further configured to determine a difficulty to intubate and / or ventilate value based on the at least one static parameter in combination with the at least one dynamic parameter.

According to another aspect of the invention, a system to determine the difficulty in ventilating a subject comprises: an imaging module to acquire image parameters, the image parameters comprising an image of part of a head or neck of a subject; a parameter extraction module configured to extract at least one parameter of the subject from the image parameters; a classification module comprising a classification algorithm, the classification algorithm being configured to determine a difficulty to ventilate value based on the at least one dynamic parameter.

In an embodiment, the imaging system is operable to determine the difficulty to mask ventilate a subject.

In an embodiment, the static parameter includes a variable related to the presence of facial hair; features used to determine whether a patient snores; absence of one or more teeth.

In an embodiment, the imaging module comprises a face structural mapping module operable to generate a digital mask of at least part of the subject, the parameter extraction unit being operable to process the digital mask to determine the at least one dynamic and / or static parameters.

In a preferred embodiment, the classification algorithm incorporates a learning algorithm , for instance Random Forest Classifier, configured to be trained on a training set comprising parameters for a number of subjects. The number of subjects of the training set is preferably at least 100.

The system may further comprise an imaging unit, the imaging unit being operable to capture a static image of a subject.

In an embodiment, the imaging unit comprises one or more cameras.

In an embodiment, the imaging unit comprises at least two cameras arranged to obtain images along intersecting axes.

The system may include a first camera arranged to obtain frontal images of a subject, and a second camera arranged to obtain profile images of a subject.

The imaging unit may advantageously further comprise a depth camera.

The invention also includes a computer-readable storage medium comprising a software code stored thereon which comprises the system described herein.

Further disclosed herein is a method of determining the difficulty in intubating and / or ventilating a subject, the method comprising operating a system to: acquire image parameters on an imaging module, the image parameters comprising a moving image of part of a head or neck of a subject; extract a plurality of parameters including at least one dynamic parameter from the image parameters using a parameter extraction module; determine a difficulty to intubate and / or ventilate value based on said plurality of parameters including the at least one dynamic parameter by processing the said plurality of parameters with a classification algorithm.

The method may also advantageously to determine the difficulty in extubating a subject, whereby the classification algorithm is configured to determine a difficulty to extubate value based on the at least one dynamic parameter.

Also disclosed herein is a method of determining the difficulty in mask ventilating a subject, the method comprising operating a system to: acquire image parameters on an imaging module, the image parameters comprising an image of part of a head or neck of a subject; extract a plurality of parameters from the image parameters using a parameter extraction module; determine a difficulty to ventilate value based on said plurality of parameters by processing the said plurality of parameters with a classification algorithm.

Also disclosed herein is a method of determining the difficulty of extubating a subject, with identification of sleep apnea syndrome.

Further objects and advantageous features of the invention will be apparent from the claims, from the detailed description, and annexed drawings.

## BRIEF DESCRIPTION OF THE DRAWINGS

For a better understanding of the invention, and to show how embodiments of the same may be carried into effect, reference will now be made, by way of example, to the accompanying diagrammatic drawings in which:

Figure 1 is a simplified schematic diagram of a system to determine the difficulty in intubating and / or ventilating a subject according to the invention;

Figure 2 is a diagram of an imaging unit of the system of figure 1;

Figure 3 shows frontal images of a face and neck of a subject with an AAM mask thereon;

Figure 4 shows frontal images of a face and neck of a subject in a state of extreme movement with an AAM mask thereon;

Figure 5 shows profile images of a face and neck of a subject with an AAM mask thereon;

Figure 6 shows profile images of a subject during a movement task, wherein dynamic features are obtained;

Figure 7 is a diagram of an optic flow method used in dynamic feature extraction;

Figure 8 is a diagram of a parameter extraction module of the system of figure 1;
Figure 9 shows an annotated profile image of a subject to show dynamic features;
Figure 10 is a ROC curve for the binary problem using feature level fusion;
Figure 11 is a ROC curve for the binary problem using decision level fusion.

## DETAILED DESCRIPTION OF EXEMPLARY EMBODIMENTS

### 1. Arrangement of system to determine the difficulty in intubating and/or ventilating and/or extubating a subject

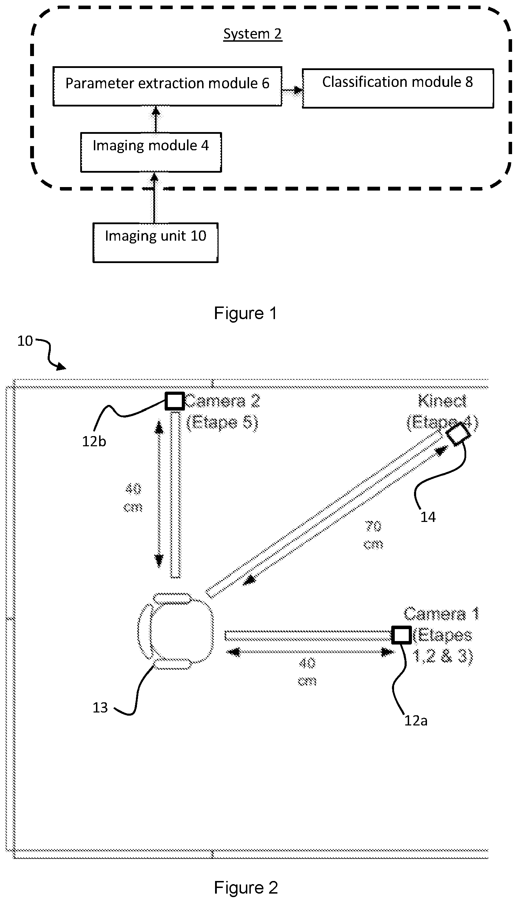

Figure 1 shows an example of a system 2 to determine the difficulty in intubating and / or ventilating a subject. The system 2 comprises an imaging module 4 configured to acquire image parameters comprising an image of part of a head or neck of a subject; a parameter extraction module 4 configured to extract parameters of the subject from the image parameters; and a classification module 8 to classify the parameters.

The imaging module 4 receives image parameters from an imaging unit 10. The imaging unit 10 comprises one or more cameras 12. Still images from the or each camera may be used to calculate the parameters discussed in section 2.2.1 and 2.2.2 below, wherein the parameters are referred to as distance features. In an advantageous embodiment one or more of the cameras 12 is operable to capture an image stream of a subject such that motion of a subject can be captured. The motion of a subject can be used to calculated dynamic parameters, which are discussed in more detail in section 2.2.3, wherein the dynamic parameters are referred to as dynamic features. In an advantageous embodiment the imaging unit comprises one or more depth cameras 14 which are used to improve the accuracy of the image parameters.

An example imaging unit 10 is shown in figure 2, wherein the cameras 12 comprise two high resolution web cameras 12a, 12b, wherein camera 12a is arranged to obtain a front image of a subject 13 and camera 12b is arranged to obtain a profile image of a subject. Hence the cameras 12a and 12b are arranged on intersecting axes, which in this example are orthogonal to each other. In this example a depth camera 14, such as a Microsoft™ Kinect is arranged to measure depth of the oropharyngeal cavity.

The parameter extraction module 6 is operable to process the image parameters, for example by means of applying a mask and extract parameters that may include dynamic parameters. The parameter extraction module 6 is discussed in more detail in section 2 below.

The classification module 8 receives the parameters from the parameter extraction module 6 and processes them by means of a classification algorithm to determine a difficulty to intubate and / or ventilate value and / or extubate. The classification algorithm is discussed in more detail in section 4 below.

### 2. Features Extraction

The target anatomical and morphological characteristics used for assessment of difficult intubation and / or ventilation and / or extubation or sleep apnea syndrome are features of the neck and the head of the patient. They include some physical and morphological characteristics that are commonly used as predictors of difficult laryngoscopy as well as other characteristics. The other characteristics are mainly some which are difficult to quantify during a normal consultation (for example area of the mouth opening).

The features are of three different kinds: distances; models' coefficients and; dynamic features. Distances and models' coefficients are obtained using active appearance models (AAM). In section 2.1 below AAM is discussed together with the associated masks. Section 2.2.1 discusses distance features. Section 2.2.2 discusses models' coefficients. Section 2.2.3 discusses dynamic features.

### 2.1 Description of Masks

Active Appearance Models (AAM) [cf C. T. G.J. Edwards, T. Cootes (1998). Interpreting face images using active appearance models. In I. C. Society (ed.), FG '98: Proceedings of the 3rd International Conference on Face and Gesture Recognition, Nara, Japan, pp. 300-305 and M. B. Stegmann (2000). Active Appearance Models: Theory, Extensions and Cases. Master's thesis, Informatics and Mathematical Modelling, Technical University of Denmark, DTU, Richard Petersens Plads, Building 321, DK-2800 Kgs. Lyngby.] automatically provide a defined set of landmarks (a mask) on an object such as a face of a subject. The landmarks' positions are described by coefficients obtained with Principal Component Analysis (PCA). Those coefficients can be seen as the deviation of the current instance of the object with respect to the mean object along one mode of variation. Mathematically, the relationship between the current instance of the object and the mean object is thus given by:

$$\mathbf{f}(\mathbf{p}) = \mathbf{f}_0 + \sum_{i=0}^{n} p_i \mathbf{b}_i$$

where $f_0$ is the mean object, $b_i$ is the ith shape basis and $p = [p1; p2; :::; pn]$ are the shape parameters.

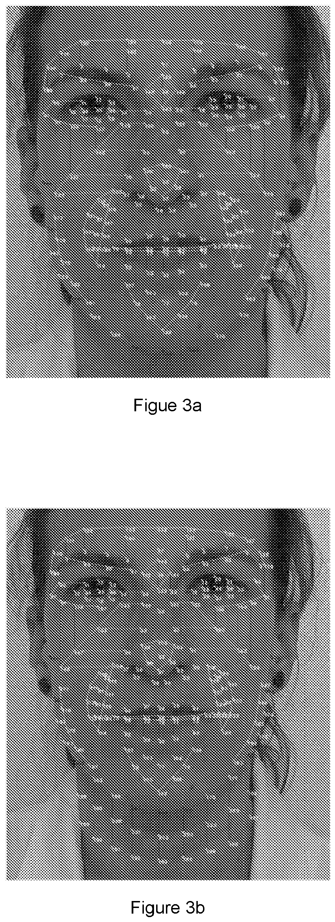

Herein a full frontal 188 points mask is used as shown in figure 3a, although it will be appreciated that other suitable masks may be used, for example, those with different numbers of points. This mask corresponds to a neutral position and neutral expression and may advantageously contain landmarks for each eyebrow, each eye, the nose, the mouth, the chin, the naso-labial furrows and various wrinkles. Various different masks described below (extreme movement masks and profile masks) are all derived from this one. This means that most of the landmarks are the same (except for the ones inside the mouth) and thus a direct correspondence can be found between points on both masks. The masks are defined in such a manner to allow tracking when the pose changes and even switching between masks for extreme pose changes.

*2.1.1 Extended mask for neck*

To assess the width of the neck, especially when the base of the neck is much larger than the top (for example in obese patients), the Fig. 3a face mask may optionally be extended by adding a number of points on the neck, for example 18 points. This results in the full frontal mask shown in figure 3b.

*2.1.2 Extreme movements: mouth wide open and tongue out*

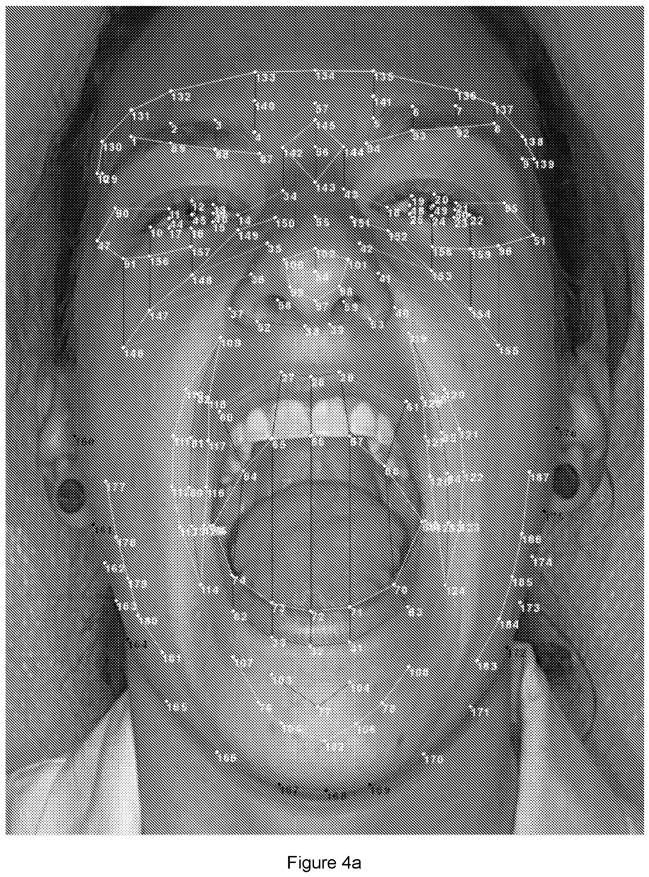

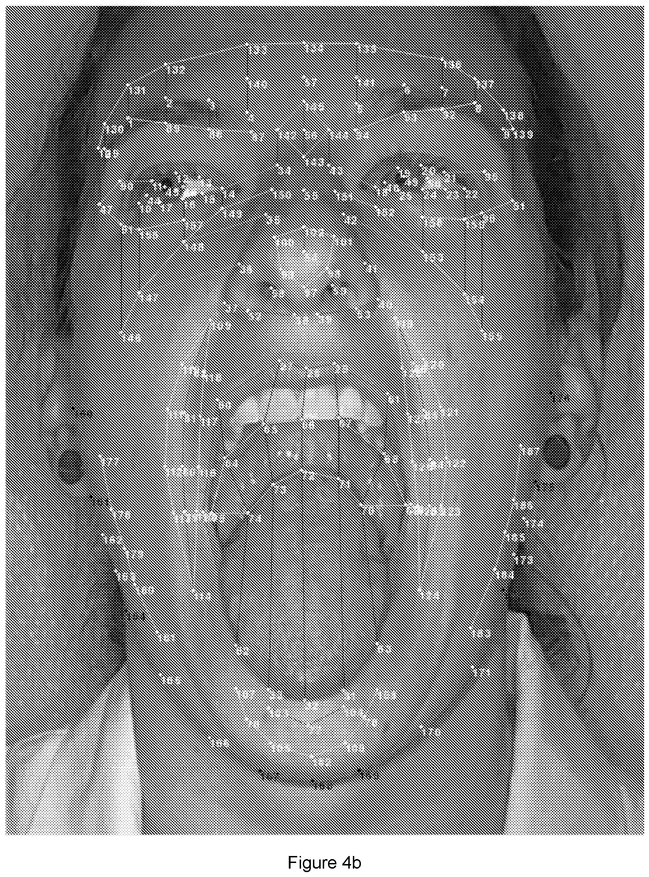

Two new models may be derived from the face mask model of Fig. 3a or 3b, or another suitable model, to handle images with extreme facial movements. An example of an extreme facial movement is a frontal image with the mouth wide open and tongue in and a frontal image with the mouth wide open and tongue out as shown in figures 4a and 4b respectively.

In the case of the mouth wide open and tongue in, in order to extract the width and the height of the mouth opening, the landmarks defining the inside of the lips (point 64 - point 75 in figure 4a) may be slightly moved. For example, they are defined such that they follow either the teeth or the lips, depending on what is present in the image. These landmarks may thus define the perimeter of the opening.

In the case of the mouth wide open and tongue out movement, ideally the same set of landmarks (point 64 - point 75 in figure 4b) is used to segment the region of interest, for example, the region which is assessed when using the Mallampati grade assessment. The region delimited by those landmarks may then be where the uvula can be found, if visible.

*2.1.3 Profile Masks*

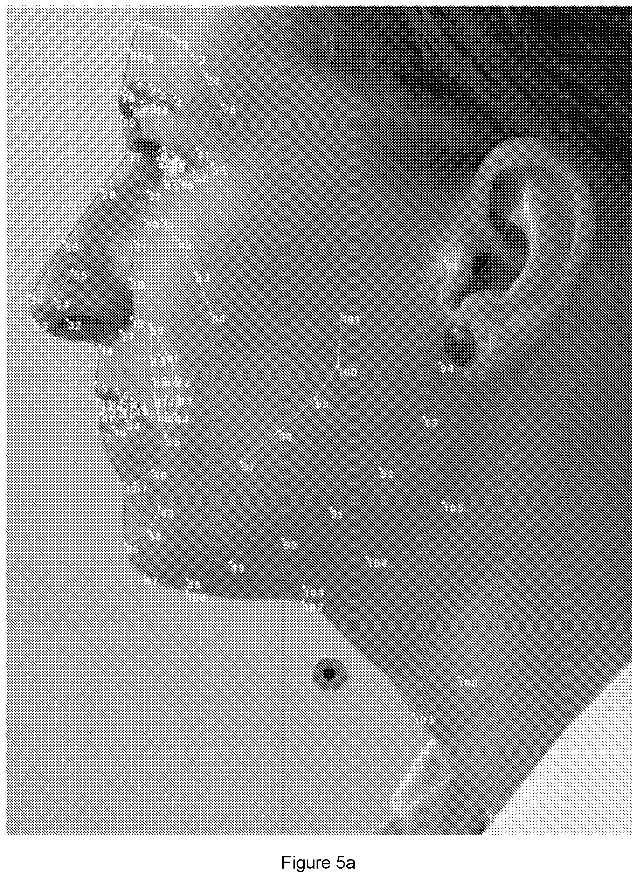

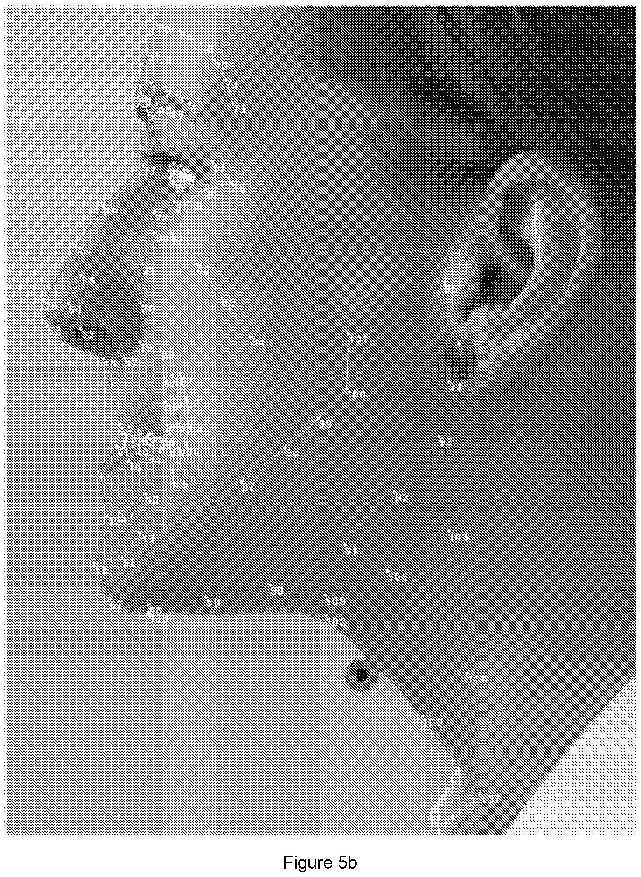

The mask that handles profile images may also derived from the generic full frontal mask, however it will be appreciated that other suitable masks may be used. It may be built by selecting all the landmarks on one half of the face (either the right or left side, for the corresponding profile) on the frontal mask and adjusting them to the profile view. Eight, or another suitable number of additional points (point 102 - point 109) may also be defined on the neck, as shown in figure 5a. The same mask may be used both for neutral expression, figure 5a and for a top lip bite test, figure 5b.

*2.2 Computation of the features*

*2.2.1 Distances*

Most of the anatomical and morphological features that need to be extracted *are the* distances between landmarks of the face and neck. The positions of the landmarks are given by the mask after fitting the AAM on the subject image. Examples of such distances are listed below.

*From the frontal image, in neutral position and using the mask of Fig. 3b :*

1. distance between the upper lip and the nose:

$$d2\_lip\text{-}nose = \left\| p_{98} - \frac{p_{28} + p_{29}}{2} \right\|$$

2. distance between the lower lip and the tip of the chin:

$$d2\_lip\text{-}chin = \| p_{108} - p_{92} \|$$

3. width of the neck:

$$w2\_neck = \| p_{172} - p_{164} \|$$

4. width of the face:

$$w2\_face = \| p_{178} - p_{160} \|$$

5. height of the face. As there are no reliable landmarks on the part of the face above the eyebrows, the height of the face may be approximated by the distance between the tip of the chin and the point between the eyes:

$$h2\_face = \left\| p_{108} - \frac{p_{34} + p_{43}}{2} \right\|$$

6. distance between the eyes:

$$d2\_eye\text{-}eye = \left\| \frac{p_{18} + p_{22}}{2} - \frac{p_{10} + p_{14}}{2} \right\|$$

*From the profile image, in neutral position and using the mask of Fig. 5a and 5b :*

1. thyromental distance in neutral position:

$$d2\_thyro\text{-}mento = \| p_{87} - p_{103} \|$$

2. distance between the angle of the mandible and the tip of the chin:

$$d2\_chin\text{-}mandib = \| p_{93} - p_{87} \|$$

3. distance between the hyoid bone and the chin:

$$d2\_mento\text{-}hyoid = \| p_{103} - p_{87} \|$$

4. distance between the hyoid bone and the thyroid cartilage:

$$d2\_thyro\text{-}hyoid = \| p_{103} - p_{100} \|$$

*From the frontal image, with mouth wide open, tongue in and using the mask of Fig. 4a, 4b :*

1. height of the mouth opening:

$$h2\_mouth = \|p_{72} - p_{68}\|$$

2. width of the mouth opening:

$$w2\_mouth = \|p_{69} - p_{75}\|$$

3. area of the mouth opening:

$$s2\_mouth = \sum_{i=0}^{8} \frac{1}{2} \|(p_b - p_a) \times (p_c - p_a)\|$$

where pa, pb and pc are the corners of each non-overlapping triangles defined by the set of landmarks p64 - p75.

Other distance features that may be measured, together with other user input, is detailed in Annex 1. When specifically considering the ease of mask ventilation, one or more of the same features as for intubation may be used however in an advantageous embodiment the following features may be measured: absence of one or more teeth, particularly the incisors canines and premolars; the presence of facial hair such as a beard or stubble; features used to determined whether a patient snores (this feature may also be input directly by an operator after asking the subject)

*2.2.2 Models' coefficients*

In addition to the above distance features that are computed from the mask, further features can be considered, such as the coefficients describing each mask. These features are given in table 1. The model's coefficients represent and describe the statistical variations of the face and its appearance. The coefficients allow to quantify morphological, structural and intrinsic variation of a patient's face otherwise impossible to describe by a human eye.

Table 1: Features and accuracies on the three-classes problem Table

|  | features | best accuracy |
|---|---|---|
| Types | 2D distances | 0.47 |
|  | 3D distances | 0.418 |
|  | AAM coefficients | 0.667 |
|  | 3D shape model coefficients Dynamic features | 0.611 |
| Modality | photo_1_front | 0.56 |
|  | feautres from photo_1_profile | 0.551 |
|  | features from photo_open_front | 0.58 |
|  | features from photo_tiree_front | 0.733 |

*2.2.3 Dynamic features*

In an advantageous embodiment dynamic features are used to describe and quantify the mobility of a body part of the subject, for example the head or neck. The dynamic features may be derived from a range of movements, for example: head extension up and down with the mouth open or closed or open with the tongue stretched out; head rotation left and right with the mouth open or closed or open with the tongue stretched out; tongue extension back and forth; lip bite.

The following describes an example comprising head extension movement of a subject with the mouth closed, however it will be appreciated that a similar technique can be applied to other movements to determine other dynamic features.

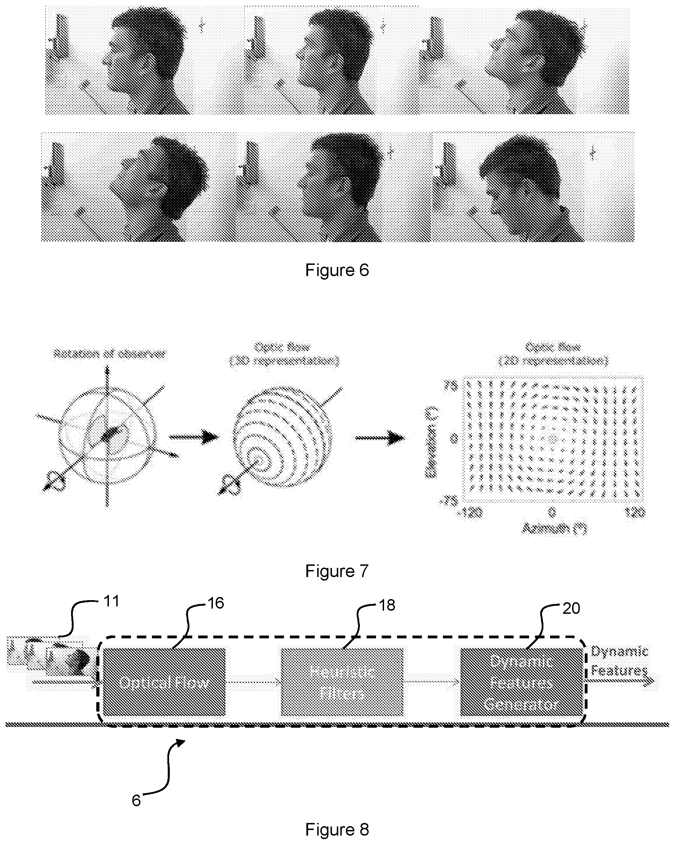

An example of head extension movement is provided in figure 6, wherein a sequence of images that are obtained from the imaging unit 10 are shown during movement. In more detail, the subject is asked to perform a movement which starts with the head in a frontal position, thereafter the head is moved up to the top and thereafter it is moved through the frontal position down to the bottom.

The development and the use of algorithms to describe motion in a scene has been a main topic in several branches of video compression, computer vision and image processing research. A suitable technique to address this problem is optical flow, which is used herein as an example algorithm to describe motion. Accordingly, it will be appreciated that other suitable algorithms may be used. The optical flow represents the apparent motion of an object in a visual scene due to the relative motion between an observer and the scene itself.

Figure 7 shows an example of the optical flow technique, wherein the rotation of an observer (in this case a fly) is captured and represented as a 3D and 2D representation. The 2D representation comprises a plot of elevation and Azmunth, wherein the optic flow at each location is represented by the direction and length of each arrow.

The concept of optical flow is described in more detail in the following references which are incorporated herein by reference: S. Baker, D. Scharstein, J. Lewis, S. Roth, M. J. Black, and R. Szeliski. A database and evaluation methodology for optical flow. In ICCV, 2007; Wedel, T. Pock, and D. Cremers. Structure- and motion-adaptive regularization for high accuracy optic flow. In ICCV, 2009; Wedel, T. Pock, C. Zach, D. Cremers, and H. Bischof. An improved algorithm for TV-L1 optical flow. In Dagstuhl Motion Workshop, 2008; M. Werlberger, W. Trobin, T. Pock, A. Wedel, D. Cremers, and H. Bischof. Anisotropic Huber-L1 optical flow. In BMVC, 2009; H. Zimmer, A. Bruhn, J. Weickert, L. Valgaerts, A. Salgado, B. Rosenhahn, and H.-P. Seidel. Complementary optic flow. In EMMCVPR, 2009; M. Sorci, Automatic face analysis in static and dynamic environments, Chapter 9, PhD. Thesis, EPFL 2009.

Herein the application of the optical flow technique is particularly convenient due to the amount of constraints of the moving object that is described (which in this example is the head of a subject). These constraints can be considered to include at least one or more of the following: the fixed position of the camera; the profile and consistent position of the subject in the scene; the known movement of the head (frontal-top-frontal-bottom); the uniform background.

The aforementioned constraints allow combining the description of the movement provided by the optical flow with a series of heuristics so as to filter the different sources of noise that occur during the video acquisition process. Examples of noise that can be filtered include shadows due to changes in lightning conditions; subtle movements of the camera; noise of the acquisition system.

Figure 8 shows an exemplary process for extracting dynamic features. Wherein a sequence of images 11 (for example those shown in of figure 6) obtained by the imaging unit 10 are sent to the imaging module 10. The imaging module 10 thereafter sends the images 11 to the parameter extraction module 6. The parameter extraction module is operable to extract the dynamic features from the images.

In this example the images are first processed by an optic flow module 16 of the extraction module 6 to map the direction and movement of the subject in 3D and 2D optic flow representations. More particularly, optic flow module 16 comprises an optical flow algorithm which processes the entire subject's motion sequence: the output of this module is a frame per frame motion field describing all movement in the scene.

The data output of the optic flow module 16 may be supplied to a filter module 18 of the extraction module 6, which process the data to remove sources of noise. The filter module 18 may comprise heuristic filter blocks which apply a set of rules and thresholds to remove any detected noise in the data. The output of the filter module 18 is a filtered version of the motion field data from the previous module.

A dynamic features generator module 20 receives the filtered data, and processes the data to quantify the movement (which in this example is head movement) throughout the entire sequence of movement. In an advantageous embodiment the generator module 20 is operable to quantify the movement by means of one or more of dynamic features, which in this example is three as described following:

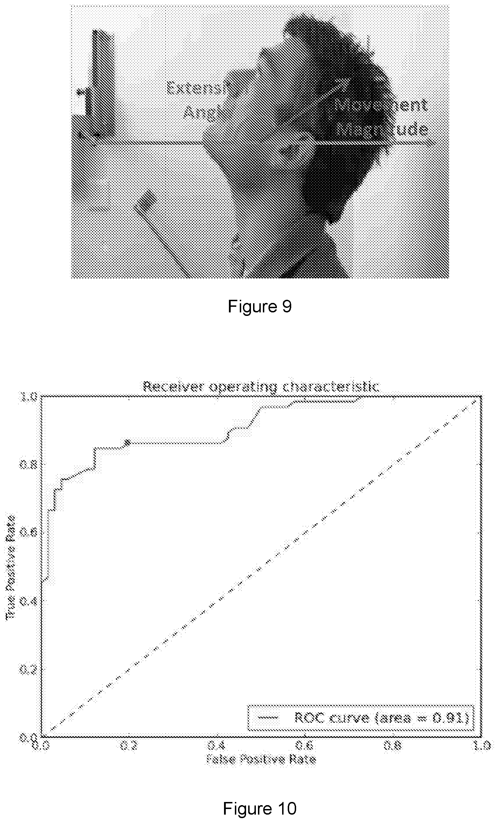

> 1. Extension angle: with reference to figure 9, this is the measure in radians of the maximum extension angle of the head and is obtained by considering average motion vector among those describing coherently the movement of the patient's head.
>
> 2. Movement Magnitude: with reference to figure 9, this measure represents the magnitude/amplitude of the motion vector used to define the extension angle. The values of this measure may be normalized values between 0 and 1.
>
> 3. Motion Coherence Index (MCI): this index complements the information on the dynamic of the head provided by the two previous measures by quantifying the coherence of the motion. This requires consideration of the movement of the head from its frontal position until his up most extended position is achieved. The coherence is then described as the ratio between the number of horizontal motion vectors in the "head blob" moving coherently in the positive x axis direction (the positive x-axis direction is shown in Figure 9) and the total number of motion vectors related to the head. This index may be normalized between 0 and 1.

*3. The multi class problem*

In a preferred embodiment three classes are defined: easy, intermediate and difficult. The classification may be based on information provided by a medical practitioner and in an advantageous embodiment relies on two commonly used classifications: the laryngoscopic view, as defined by Cormack and Lehane, and the intubation difficulty scale (IDS) defined by Adnet et al, however it will be appreciated that other suitable classifications may be used.

The extracted features that were discussed in section 2 may be grouped into subsets according either to their type and / or to the image they are computed from. Complete sets of features may also be considered. An example of how the features may be classified is presented as follows.

1. Ranking of the features: A Random Forest classifier may be trained on the complete subset of features and its parameters (n_estimator: number of trees and max_features: number of features to randomize when looking for the best split) are optimized for accuracy using 5 folds cross-validation.

2. Feature selection: Based on the ranking, an increasing number of the best features may be selected to constitute a subset. A classifier of which parameters are not optimized (n_estimator=200 and max_features=p N features) is trained and tested on each subset preferably using 5 folds cross-validation. The subset on which the accuracy is the higher may then be kept as the optimal subset.

3. For each optimal subset, a Random Forest classifier may again optimized following the same process as in 1 above but on a different subset of features. The classifier trained with the best set of parameters (according to its accuracy) may then be saved.

An example of different optimal subsets of features is given in the appended Example A. Once the best subsets are determined for each initial subset of features and that the corresponding optimized classifier are available, two different methods can be used to fuse the results of those sub-classifier: Feature-level fusion or Decision-level fusion.

*3.1 Feature-level fusion*

In an advantageous embodiment an overall accuracy of 72.2% was obtained and the confusion matrix is reported in table 2 below The confusion matrix is a specific table layout that allows visualization of the performance of an algorithm, typically a supervised learning one. Each column of the matrix represents the instances in a predicted class, while each row represents the instances in an actual class. The name stems from the fact that it makes it easy to see if the system is confusing two classes (i.e. commonly mislabeling one as another).

| | | Predicted cl. | |
|---|---|---|---|
| | easy | interm. | diff. |
| easy | 54 | 8 | 8 |
| intermediate | 13 | 41 | 12 |
| difficult | 7 | 11 | 48 |

Table 2: Confusion matrix for 3 classes, feature-level fusion problem

As the detection of the difficult class is of most interest, in an advantageous embodiment the easy and intermediate classes are grouped a-posteriori in order to compute the statistics of the classification for the difficult class against the other. In an example embodiment, this results in the confusion matrix presented in table 3 below.

| | Predicted | | |
|---|---|---|---|
| | difficult | rest | |
| difficult | 48 | 18 | sensitivity=72.7% |
| rest | 16 | 116 | specificity=87.9% |
| | PPV=75% | NPV=86.6% | |

Table 3: Confusion matrix for the *difficult* class against the rest

*3.2 Decision-level fusion*

In an advantageous embodiment, each individual classifier is used to output the probabilities that the sample belongs to each class. Those probabilities may then be weighted and summed. Finally, the argmax() may be used to take the decision. The weights are computed from the scaled accuracies (given in table 1) of each individual classifier as:

$$w = \frac{1}{1 - 0.9 \cdot accuracy_{[0..9]}} \qquad (2)$$

where

$$accuracy_{[0..9]} = \frac{accuracy - \min(accuracies)}{\max(accuracies) - \min(accuracies)} \qquad (3)$$

In an advantageous embodiment an overall accuracy of 71.7% is obtained and the confusion matrix as shown table 4 below:

| | | Predicted cl. | | |
|---|---|---|---|---|
| | | easy | interm. | diff. |
| Actual cl. | easy | 54 | 9 | 3 |
| | intermediate | 10 | 42 | 14 |
| | difficult | 8 | 12 | 46 |

Table 4: Confusion matrix for 3 classes, decision-level fusion problem

Again, the easy and intermediate classes may be grouped a-posteriori in order to compute the statistics of the classification for the difficult class against the other. This results in the confusion matrix presented in table 5.

| | | Predicted | | |
|---|---|---|---|---|
| | | difficult | rest | |
| Actual | difficult | 46 | 20 | sensitivity=69.7% |
| | rest | 17 | 115 | specificity=87.1% |
| | | PPV=73.0% | NPV=85.2% | |

Table 5: Confusion matrix for the *difficult* class against the rest

*4. The simplified binary problem*

The following simplified binary problem may also be considered with the following classes: easy and difficult. The method and the features are the same as before. In an advantageous embodiment the individual accuracies obtained for each sub-classifiers are reported in table 6. As in the above, Feature-level fusion and Decision-level fusion may advantageously be used.

Table 6: features and accuracies on the binary problem

|  | features | best accuracy |
|---|---|---|
| Types | 2D distances | 0.689 |
|  | 3D distances | 0.682 |
|  | AAM coefficients | 0.879 |
|  | 3D shape model coefficients | 0.871 |
|  | Dynamic features | 0.554 |
| Modality | feateres from photo_1_front | 0.795 |
|  | features from photo_1_profile | 0.734 |
|  | features from photo_open_front | 0.864 |
|  | features from photo_tiree_front | 0.856 |

*4.1 Feature-level fusion*

In an advantageous embodiment an overall accuracy of 86.4% was obtained and the confusion matrix is as shown in table 7 below. As the problem is a binary classification problem, a receiver operating characteristic (ROC) curve can be derived as shown in figure 10. As an example, the marked point on the curve corresponds to a True Positive Rate of 86% for a False Positive Rate of 20%.

|  | Predicted | | |
|---|---|---|---|
|  | difficult | easy | |
| Actual difficult | 56 | 10 | sensitivity=84.9% |
| easy | 8 | 58 | specificity=87.9% |
|  | PPV=87.5% | NPV=85.3% | |

Table 7: Confusion matrix for the *difficult* class against the *easy* one

*4.2 Decision-level fusion*

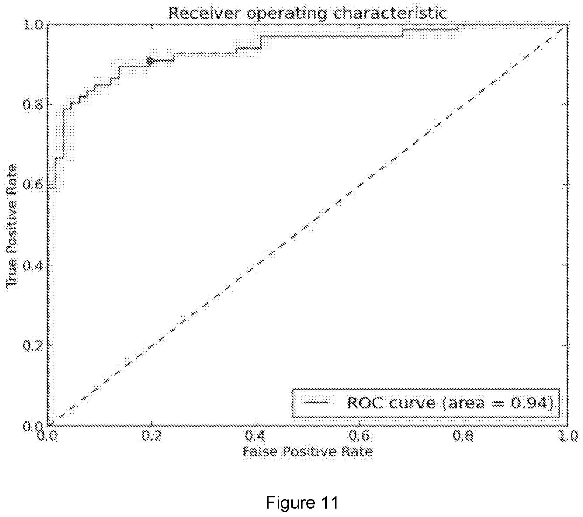

In an advantageous embodiment an overall accuracy of 87.9% was obtained and the confusion matrix is as shown in table 8 below. The associated ROC curve is as shown in figure 11. As an example, the marked point on the curve corresponds to a True Positive Rate of 91% for a False Positive Rate of 20%.

The output of the system is the class of difficult intubation or ventilation the patient is recognized by the system to belong to. In case of the 3-class approach the patient will be classified in one of the aforementioned classes: easy, intermediate or difficult. In the binary approach the 2 classes will be: easy or difficult.

*Example A: Optimal subsets of features for the multiclass problem*

A.1 ALL

When performing feature selection over the whole set of features, 33 features are selected out of 373:

- 10 coefficients from the mask fitted on the patient's image with the mouth open and the tongue extended out (coefficients 1, 2, 22, 23, 29, 33, 34, 37, 49, 53);
- 5 coefficients from the mask fitted on the frontal patient's image (coefficients 5, 18, 27, 36, 39);
- 4 coefficients from a smaller sub-model of the mouth fitted on the patient's image with the mouth open and the tongue extended out (coefficients 0, 4, 28,41);
- 3 coefficients of a 3D shape model fitted on the patient's image with the mouth open and the tongue extended out (coefficient 6, 12, 14);
- 3 coefficients from the mask fitted on patient's image with the mouth open (coefficients 13, 36, 41);
- 1 coefficient from the mask fitted on profile patient's image (coefficient 13);
- the surface of the opening of the mouth, computed from the 3D shape model;

- the following 2D distances: d2_lip-nose, h2_face, w2_face, w2_mouth, w2_neck;
- the maximum angle of extension, estimated with the optical flow method;

### A.2 2D_DISTANCES

When performing feature selection on the subset of features that are 2D distances, 9 features are selected out of 12: d2_lip-nose, h2_mouth, h2_face, d2_thyro-hyoid, d2_lip-chin, s2_mouth, w2_mouth, w2_face, d2_mento-hyoid.

### A.3 3D_DISTANCES

When performing feature selection on the subset of features that are 3D distances, 4 features are selected out of 12: w3_mouth, d3_lip-nose, s3_mouth, w3_face.

### A.4 AAM_COEFFS

When performing feature selection on the subset of features that are AAM coefficients, 70 coefficients are selected out of 222.

### A.5 3D_SHAPE_COEFFS

When performing feature selection on the subset of features that are 3D shape model coefficients, 20 coefficients are selected out of 72.

### A.6 DYNAMIC

When performing feature selection on the subset of features that are dynamic features, 2 features are selected out of 3: Max Angle and Max RAD Norm.

*Example B: Statistical terms*

A reminder of the statistical terms is provided here after.
True positive = a difficult intubation that had been predicted to be difficult.
False positive = an easy intubation that had been predicted to be difficult. (Type I Error, or false alarm)
True negative = an easy intubation that had been predicted to be easy.
False negative = a difficult intubation that had been predicted to be easy. (Type II Error, or miss)
Sensitivity = the percentage of correctly predicted difficult intubations as a proportion of all intubations that were truly difficult, also called True Positive Rate, or Recall i.e.:

$$\text{Sensitivity} = \frac{\text{true positives}}{(\text{true positives} + \text{false negatives})}$$

Specificity = the percentage of correctly predicted easy intubations as a proportion of all intubations that were truly easy, also called True Negative Rate i.e.:

$$\text{Specificity} = \frac{\text{true negatives}}{(\text{true negatives} + \text{false positives})}$$

Positive predictive value = the percentage of correctly predicted difficult intubations as a proportion of all predicted difficult intubations, also called Precision i.e.:

$$\text{Positive predictive value} = \frac{\text{true positives}}{(\text{true positives} + \text{false positives})}$$

Negative predictive value = the percentage of correctly predicted easy intubations as a proportion of all predicted easy intubations, i.e.:

$$\text{Negative predictive value} = \frac{\text{true negatives}}{(\text{true negatives} + \text{false negatives})}$$

Accuracy = the percentage of correctly predicted easy or difficult intubations as a proportion of all intubations, i.e.:

$$\text{Accuracy} = \frac{(\text{true positives} + \text{true negatives})}{(\text{true positives} + \text{true negatives} + \text{false positives} + \text{false negatives})}$$

False positive rate, a:

$$\alpha = 1 - \text{Specificity} = \frac{\text{false positive}}{\text{false positive} + \text{true negative}}$$

False negative rate, $\beta$:

$$\beta = 1 - \text{Sensitivity} = \frac{\text{false negative}}{\text{true positive} + \text{false negative}}$$

*Example C: Assessment of difficulty to intubate using 5 static images and 3 dynamic sequences*

The above method was applied using the imaging unit 10 arrangement of figure 2. Statistical tests used were median or Chi-square when appropriate. Data was analysed using the JMP 6 statistical package (SAS Institue Inc., Cary, NC, USA). The Chi2 test was used for comparing categorical variables. Student t -test was used for comparing normally distributed continuous variables. Pearson correlations were used to examine the association between continuous variables. A p value < 0.05 was considered statistically significant.

During the study period, a training set number of patients with the demographic data shown in the table 1, were entered in the program.

**Table 1. Patient's demographics**

|  | Mean | SD |  |
|---|---|---|---|
| **Age [years]** |  |  |  |
| **Height [cm]** |  |  |  |
| **Weight [Kg]** |  |  |  |
| **Male / Female** |  |  |  |

A total of 5 static images and 3 dynamic sequences as shown in table 2 below, were collected per patient:

Table 2. Patient's demographics

|  |  | Type of images / Camera | Head position |
|---|---|---|---|
| 1 | **Frontal and Profile still images** | **Static / Webcam** | **Neutral head position**<br>**a. mouth closed**<br>**b. mouth open, tongue in**<br>**c. mouth open, tongue stretched out**<br>**d. upper lip biting test** |
| 2 | **Frontal and Profile videos** | **Dynamic / Webcam** | **Maximum Right and Left Head rotation** |

(continued)

| | | Type of images / Camera | Head position |
|---|---|---|---|
| 3 | Frontal and Profile videos | Dynamic /Webcam | Maximum Head Extension and Flexion, a. mouth closed b. mouth open, tongue stretched out |
| 4 | Frontal still images | Static / Kinect® | Neutral head position Mouth open, tongue stretched out |

The static neutral position mask models Fig. 4a, 4b and Fig. 3b were used to provide anatomical and facial landmarks on the patient population. Table 3 below shows the types of metadata of patients dataset that was collected.

Table 3 : metadata of patients dataset

| Feature [mm] | mean | STD | Range |
|---|---|---|---|
| d lip-nose | | | |
| d lip-chin | | | |
| w neck | | | |
| w face | | | |
| h face | | | |
| d eye-eye | | | |
| d thyro-mento n | | | |
| d chin-mandib | | | |
| d mento-hyoid n | | | |
| d thyro-hyoid n | | | |
| d nose-chin | | | |
| h mouth | | | |
| s mouth | | | |
| l incisors | | | |
| Shape tongue | | | |
| d hyoid-mental | | | |
| d thyro-hyoid | | | |

*Mallampati evaluation*

The AAM fits well to the Mallampati classification case, not only because it efficiently segments the object and models the shape and texture variations among different subjects but it also includes certain preprocessing steps such as shape alignment and texture warping which make us invariant to factors like translation, rotation and scaling. Automatic identification and recording of 177 facial points were collected on each patients face.

The Mallampati classification correlates tongue size to pharyngeal size (Mallampati SR, Gatt SP, Gugino LD, Waraksa B, Freiburger D, Liu PL. A Clinical sign to predict difficult intubation; A prospective study. Can Anaesth Soc J 1985; 32: 429-434.). This test is performed with the patient in the sitting position, head in a neutral position, the mouth wide open and the tongue protruding to its maximum. Patient is asked not to phonate as it can result in contraction and elevation of the soft palate leading to a spurious picture. Original classification is assigned according to the extent the base of tongue is able to mask the visibility of pharyngeal structures into three classes. In Samsoon and Young's modification (Samsoon GLT, Young JRB. Difficult tracheal intubation : a retrospective study. Anaesthesia 1987; 42: 487-490.) of the Mallampati classification, a IV class was added.

The view is graded as follows : class I, soft palate, fauces, uvula, and pillars are visible; class II, soft palate, fauces, and uvula are visible; class III, soft palate and base of the uvula are visible; class IV, soft palate is not visible at all.

A front view picture of the patient's face with mouth wide open and tongue protruding to its maximum is obtained. As the Mallampati classification depends highly on the angle of view of the mouth, a video recording is then performed with the patient asked to shortly and slowy extend and flex the head to improve the view of the oro-pharynx. The images were taken by trained staff such that the head is positioned to obtain the best visibility of the oropharyngeal features. Once we obtained an accurate image based Mallampati classification, videos of patients were used to classify each frame and assess the lowest score obtained in the video, which corresponds to the optimal view, for that patient.

*Human classification:*

The assessment of the ground truth for the modified Mallampati score was separately performed by two experienced anesthesiologists only on the basis of these images. The dataset used was composed of 100 images of different subjects, equally balanced between Mallampati classes. Only the patients who were rated with a similar Mallampati classification by the two anesthesiologists were submitted to automatic analysis.

*Annex 1: Features and tests that may be used by the system*

- measurement of weight, height and age

- distance between the upper lip and the nose (point between the nostrils)

- distance between the lower lip and the tip of the chin

- width of the neck

- width of the face

- height of the face

- distance between the eyes

- angle of maximum lateral rotation of the head

- angle of maximum flexion of the neck (head against the chest)

- angle of maximum extension of the neck

- height of the mouth opening: distance between the upper and lower incisors or lips, according to what is visible on the image.

- width of the mouth opening

- area of the mouth opening

- length of the upper incisors: should allow to detect prominent incisors.

- depth of the oral cavity: distance between the lower incisors and the end of the oro-pharynx.

- Mallampati score (cf. section 2.2.2)

- shape of the back of the tongue

- thyromental distance (with the head in full extension)

- ratio of height to thyromental distance

- thyromental distance in neutral position

- distance between the corner of the mandible and the tip of the chin

- distance between the hyoid bone and the chin

- distance between the hyoid bone and the thyroid cartilage

- distance of the maximal forward protrusion of the lower incisors beyond the upper incisors

- Mallampati score

- Distance Thyro-mentale

- Distance Sterno-mentale

- Distance inter-dents (Mouth opening)

- Dents proéminentes

- Upper lip bite test

**Claims**

1. A system to determine the difficulty in intubating and / or ventilating and / or extubating a subject, the system comprising:

    an imaging module to acquire image parameters comprising a dynamic image of part of a head or neck of a subject;
    a parameter extraction module configured to extract at least one dynamic parameter from the image parameters;
    a classification module comprising a classification algorithm configured to determine a difficulty to intubate and / or ventilate value based on the at least one dynamic parameter, **characterized in that**
    the imaging module being configured to acquire image parameters comprising a head movement sequence about a neck of a subject and the dynamic parameter is determined from the head and / or neck movement,
    the head movement about the neck comprising one or more of the following movements: a head up and / or a head down movement; a head rotation to the left and / or a head rotation to the right movement; a head translational movement and / or a head arcing movement,
    wherein the imaging module is configured to acquire image parameters comprising one or more of the following movements: a jaw movement; a tongue movement; lip bite movement,
    and wherein the parameter extraction module comprises an optic flow module for processing the dynamic parameters, the optic flow module to process the image parameters to map the direction and movement of the subject in 3D and / or 2D optic flow representations.

2. The system according to claim 1, further comprising an imaging unit, the imaging unit being operable to capture a moving image of a subject.

3. The system according to claim 1 or 2, wherein the parameter extraction module comprises a filter module, the filter module to process the optic flow data to remove sources of noise.

4. The system according to any preceding claim, wherein the parameter extraction module comprises a dynamic parameter determination module, the dynamic parameter determination module to determine one or more dynamic parameters from the optic flow data.

5. The system according to any preceding claim, wherein the movement being captured is a head up and / or a head down movement and the dynamic parameter comprises one or more of the following: extension angle; movement magnitude; motion coherence index.

6. The system according to any preceding claim, wherein the imaging system is further operable to provide data comprising a static image of part of a head or neck of a user;

the parameter extraction module being further configured to extract at least one static parameter from the static image parameters;

the classification algorithm being further configured to determine a difficulty to intubate and / or ventilate value based on the at least one static parameter in combination with the at least one dynamic parameter.

7. The system according to claim 2, wherein the imaging unit comprises at least two cameras arranged to obtain images along intersecting axes.

8. The system according to the preceding claim, wherein a first camera is arranged to obtain frontal images of a subject, and a second camera is arranged to obtain profile images of a subject.

9. The system according to any one of claims 2, 7 or 8, wherein the imaging unit further comprises a depth camera.

10. A method of determining the difficulty in intubating and / or ventilating a subject, the method comprising operating a system to:

acquire image parameters on an imaging module, the image parameters comprising a moving image of part of a head or neck of a subject;

extract a plurality of parameters including at least one dynamic parameter from the image parameters using a parameter extraction module;

determine a difficulty to intubate and / or ventilate value based on said plurality of parameters including the at least one dynamic parameter by processing the said plurality of parameters with a classification algorithm, **characterized in that**

the imaging module acquiring image parameters comprising a head movement sequence about a neck of a subject and the dynamic parameter is determined from the head and / or neck movement,

the head movement about the neck comprising one or more of the following movements: a head up and / or a head down movement; a head rotation to the left and / or a head rotation to the right movement; a head translational movement and / or a head arcing movement,

wherein the imaging module further acquires image parameters comprising one or more of the following movements: a jaw movement; a tongue movement; lip bite movement,

and wherein the parameter extraction module comprises an optic flow module for processing the dynamic parameters, the optic flow module processing the image parameters to map the direction and movement of the subject in 3D and / or 2D optic flow representations.

11. The method as claimed in the proceeding claim, wherein the system comprises the features of any one of claims 1-9.

**Patentansprüche**

1. System zur Bestimmung der Schwierigkeit bei der Intubation und/oder Beatmung und/oder Extubation einer Person, wobei das System umfasst:

ein Bildgebungsmodul zum Erfassen von Bildparametern, die ein dynamisches Bild eines Teils eines Kopfes oder Halses einer Person umfassen;

ein Parameterextraktionsmodul, das ausgestaltet ist, um mindestens einen dynamischen Parameter von den Bildparametern zu extrahieren;

ein Einstufungsmodul, das einen Einstufungsalgorithmus umfasst, der ausgestaltet ist, um einen Intubations- und/oder Beatmungsschwierigkeitswert basierend auf dem mindestens einen dynamischen Parameter zu bestimmen, **dadurch gekennzeichnet, dass** das Bildgebungsmodul ausgestaltet ist, um Bildparameter zu erfassen, die eine Kopfbewegungsfolge um einen Hals einer Person umfassen, und der dynamische Parameter von der Kopf- und/oder Halsbewegung bestimmt wird,

die Kopfbewegung um den Hals eine oder mehrere von den folgenden Bewegungen umfasst: eine Kopfaufwärts- und/oder eine Kopfabwärtsbewegung; eine Bewegung für eine Kopfdrehung nach links und/oder eine Kopfdrehung nach rechts; eine Kopftranslationsbewegung und/oder eine bogenförmige Kopfbewegung,

wobei das Bildgebungsmodul ausgestaltet ist, um Bildparameter zu erfassen, die eine oder mehrere von den folgenden Bewegungen umfassen: eine Kieferbewegung; eine Zungenbewegung; Lippenbeißbewegung,

und wobei das Parameterextraktionsmodul ein optisches Flussmodul zum Verarbeiten der dynamischen Parameter umfasst, wobei das optische Flussmodul zum Verarbeiten der Bildparameter zum Abbilden der Richtung und Bewegung der Person in optischen 3D- und/oder 2D-Flussdarstellungen bestimmt ist.

2. System nach Anspruch 1, das ferner eine Bildgebungseinheit umfasst, wobei die Bildgebungseinheit betriebsfähig ist, um ein bewegtes Bild einer Person aufzunehmen.

3. System nach Anspruch 1 oder 2, wobei das Parameterextraktionsmodul ein Filtermodul umfasst, wobei das Filtermodul zum Verarbeiten der optischen Flussdaten zum Entfernen von Rauschquellen bestimmt ist.

4. System nach einem der vorhergehenden Ansprüche, wobei das Parameterextraktionsmodul ein dynamisches Parameterbestimmungsmodul umfasst, wobei das dynamische Parameterbestimmungsmodul zum Bestimmen von einem oder mehreren dynamischen Parametern von den optischen Flussdaten bestimmt ist.

5. System nach einem der vorhergehenden Ansprüche, wobei die Bewegung, die aufgenommen wird, eine Kopfaufwärts- und/oder Kopfabwärtsbewegung ist und der dynamische Parameter eines oder mehrere von Folgendem umfasst: Streckungswinkel; Bewegungsausmaß; Bewegungskohärenzindex.

6. System nach einem der vorhergehenden Ansprüche, wobei das Bildgebungssystem ferner betriebsfähig ist, um Daten bereitzustellen, die ein statisches Bild eines Teils eines Kopfes oder Halses eines Benutzers umfassen; wobei das Parameterextraktionsmodul ferner ausgestaltet ist, um mindestens einen statischen Parameter von den statischen Bildparametern zu extrahieren; der Einstufungsalgorithmus ferner ausgestaltet ist, um einen Intubations- und/oder Beatmungsschwierigkeitswert basierend auf dem mindestens einen statischen Parameter in Kombination mit dem mindestens einen dynamischen Parameter zu bestimmen.

7. System nach Anspruch 2, wobei die Abbildungseinheit mindestens zwei Kameras umfasst, die angeordnet sind, um Bilder entlang sich schneidender Achsen zu erhalten.

8. System nach dem vorhergehenden Anspruch, wobei eine erste Kamera angeordnet ist, um Frontalbilder einer Person zu erhalten, und eine zweite Kamera angeordnet ist, um Profilbilder einer Person zu erhalten.

9. System nach einem der Ansprüche 2, 7 oder 8, wobei die Bildgebungseinheit ferner eine Tiefenkamera umfasst.

10. Verfahren zum Bestimmen der Schwierigkeit beim Intubieren und/oder Beatmen einer Person, wobei das Verfahren das Betreiben eines Systems umfasst zum:

Erfassen von Bildparametern auf einem Bildgebungsmodul, wobei die Bildparameter ein bewegtes Bild eines Teils eines Kopfes oder Halses einer Person umfassen;
Extrahieren einer Vielzahl von Parametern, die mindestens einen dynamischen Parameter umfassen, von den Bildparametern unter Verwendung eines Parameterextraktionsmoduls;
Bestimmen eines Intubations- und/oder Beatmungsschwierigkeitswerts basierend auf der Vielzahl von Parametern, die den mindestens einen dynamischen Parameter umfassen, durch Verarbeiten der Vielzahl von Parametern mit einem Einstufungsalgorithmus;
**dadurch gekennzeichnet, dass**
das Bildgebungsmodul Bildparameter erfasst, die eine Kopfbewegungsfolge um einen Hals einer Person umfassen, und der dynamische Parameter von der Kopf- und/oder Halsbewegung bestimmt wird,
wobei die Kopfbewegung um den Hals eine oder mehrere von den folgenden Bewegungen umfasst: eine Kopfaufwärts- und/oder eine Kopfabwärtsbewegung; eine Bewegung für eine Kopfdrehung nach links und/oder eine Kopfdrehung nach rechts; eine Kopftranslationsbewegung und/oder eine bogenförmige Kopfbewegung,
wobei das Bildgebungsmodul ferner Bildparameter erfasst, die eine oder mehrere von den folgenden Bewegungen umfassen: eine Kieferbewegung; eine Zungenbewegung; Lippenbeißbewegung,
und wobei das Parameterextraktionsmodul ein optisches Flussmodul zum Verarbeiten der dynamischen Parameter umfasst, wobei das optische Flussmodul die Bildparameter zum Abbilden der Richtung und Bewegung der Person in optischen 3D- und/oder 2D-Flussdarstellungen verarbeitet.

11. Verfahren nach einem der vorhergehenden Ansprüche, wobei das System die Merkmale nach einem der Ansprüche 1 bis 9 umfasst.

**Revendications**

1. Système pour déterminer la difficulté d'intubation et/ou de ventilation et/ou d'extubation d'un sujet, le système comprenant :

   un module de formation d'image pour acquérir des paramètres d'image comprenant une image dynamique d'une partie d'une tête ou d'un cou d'un sujet ;
   un module d'extraction de paramètre configuré pour extraire au moins un paramètre dynamique des paramètres d'image ;
   un module de classification comprenant un algorithme de classification configuré pour déterminer une valeur de difficulté d'intuber et/ou de ventiler sur la base dudit au moins un paramètre dynamique, **caractérisé en ce que** le module de formation d'image est configuré pour acquérir des paramètres d'image comprenant une séquence de mouvements de tête autour d'un cou d'un sujet et le paramètre dynamique est déterminé à partir du mouvement de tête et/ou de cou,
   le mouvement de tête autour du cou comprend un ou plusieurs des mouvements suivants : un mouvement de tête vers le haut et/ou de tête vers le bas ; un mouvement de rotation de tête vers la gauche et/ou de rotation de tête vers la droite ; un mouvement de translation de tête et/ou un mouvement en arc de tête,
   dans lequel le module de formation d'image est configuré pour acquérir des paramètres d'image comprenant un ou plusieurs des mouvements suivants : un mouvement de mâchoire ; un mouvement de langue ; un mouvement de morsure de lèvre, et dans lequel le module d'extraction de paramètre comprend un module de flux optique pour traiter les paramètres dynamiques, le module de flux optique servant à traiter les paramètres d'image pour mapper la direction et le mouvement du sujet dans des représentations de flux optique 3D et/ou 2D.

2. Système selon la revendication 1, comprenant en outre une unité de formation d'image, l'unité de formation d'image pouvant être utilisée pour capturer une image animée d'un sujet.

3. Système selon la revendication 1 ou 2, dans lequel le module d'extraction de paramètre comprend un module de filtrage, le module de filtrage servant à traiter les données de flux optique pour retirer les sources de bruit.

4. Système selon l'une quelconque des revendications précédentes, dans lequel le module d'extraction de paramètre comprend un module de détermination de paramètre dynamique, le module de détermination de paramètre dynamique servant à déterminer un ou plusieurs paramètres dynamiques à partir des données de flux optique.

5. Système selon l'une quelconque des revendications précédentes, dans lequel le mouvement qui est capturé est un mouvement de tête vers le haut et/ou de tête vers le bas et le paramètre dynamique comprend un ou plusieurs des éléments suivants : un angle d'extension ; une amplitude de mouvement ; un indice de cohérence de mouvement.

6. Système selon l'une quelconque des revendications précédentes, dans lequel le système de formation d'image peut en outre être utilisé pour fournir des données comprenant une image statique d'une partie d'une tête ou d'un cou d'un utilisateur ;
   le module d'extraction de paramètre étant en outre configuré pour extraire au moins un paramètre statique des paramètres d'image statique ;
   l'algorithme de classification étant en outre configuré pour déterminer une valeur de difficulté d'intuber et/ou de ventiler sur la base dudit au moins un paramètre statique en combinaison avec ledit au moins un paramètre dynamique.

7. Système selon la revendication 2, dans lequel l'unité de formation d'image comprend au moins deux caméras agencées pour obtenir des images le long d'axes qui se croisent.

8. Système selon la revendication précédente, dans lequel une première caméra est agencée pour obtenir des images frontales d'un sujet, et une deuxième caméra est agencée pour obtenir des images de profil d'un sujet.

9. Système selon l'une quelconque des revendications 2, 7 ou 8, dans lequel l'unité de formation d'image comprend en outre une caméra de profondeur.

10. Procédé de détermination de la difficulté d'intubation et/ou de ventilation d'un sujet, le procédé comprenant la mise en œuvre d'un système pour :

acquérir des paramètres d'image sur un module de formation d'image, les paramètres d'image comprenant une image animée d'une partie d'une tête ou d'un cou d'un sujet ;

extraire une pluralité de paramètres comprenant au moins un paramètre dynamique des paramètres d'image en utilisant un module d'extraction de paramètre ;

déterminer une valeur de difficulté d'intuber et/ou de ventiler sur la base de ladite pluralité de paramètres comprenant ledit au moins un paramètre dynamique en traitant ladite pluralité de paramètres avec un algorithme de classification, **caractérisé en ce que**

le module de formation d'image acquiert des paramètres d'image comprenant une séquence de mouvements de tête autour d'un cou d'un sujet et le paramètre dynamique est déterminé à partir du mouvement de tête et/ou de cou,

le mouvement de tête autour du cou comprend un ou plusieurs des mouvements suivants : un mouvement de tête vers le haut et/ou de tête vers le bas ; un mouvement de rotation de tête vers la gauche et/ou de rotation de tête vers la droite ; un mouvement de translation de tête et/ou un mouvement en arc de tête,

dans lequel le module de formation d'image acquiert en outre des paramètres d'image comprenant un ou plusieurs des mouvements suivants : un mouvement de mâchoire ; un mouvement de langue ; un mouvement de morsure de lèvre,

et dans lequel le module d'extraction de paramètre comprend un module de flux optique pour traiter les paramètres dynamiques, le module de flux optique traitant les paramètres d'image pour mapper la direction et le mouvement du sujet dans des représentations de flux optique 3D et/ou 2D.

**11.** Procédé selon la revendication précédente, dans lequel le système comprend les caractéristiques de l'une quelconque des revendications 1 à 9.

System 2

Parameter extraction module 6 → Classification module 8

Imaging module 4

Imaging unit 10

Figure 1

10

Camera 2
(Etape 5)

12b

40
cm

Kinect
(Etape 4)

14

70
cm

Camera 1
(Etapes
1,2 & 3)

40
cm

13

12a

Figure 2

Figue 3a

Figure 3b

Figure 4a

Figure 4b

Figure 5a

Figure 5b

Figure 6

Figure 7

Figure 8

Figure 9

Figure 10

Figure 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8460215 B

**Non-patent literature cited in the description**

- **A. LEE ; L. T. Y. FAN ; T. GIN ; M. K. KARMAKAR ; W. D. N. KEE.** A Systematic Review (Meta-Analysis) of the Accuracy of the Mallampati Tests to Predict the Difficult Airway. *Anaesthesia & Analgesia,* June 2006, vol. 102, 1867-1878
- **MALLAMPATI, S. R. et al.** *Can. Anaesth. Soc. J.,* 1985, vol. 32, 429-34
- **SAMSOON, G. L. et al.** *Anaesthesia,* 1987, vol. 42, 487-90
- **P. A. BAKER ; A. DEPUYDT ; J. M. D. THOMPSON.** Thyromental distance measurement - fingers don't rule. *Anaesthesia,* August 2009, vol. 64, 878-882
- *Anesthesiology,* 2005, vol. 103, 429-437
- Interpreting face images using active appearance models. **C. T. G.J. EDWARDS ; T. COOTES.** FG '98: Proceedings of the 3rd International Conference on Face and Gesture Recognition. 1998, 300-305
- Active Appearance Models: Theory, Extensions and Cases. **M. B. STEGMANN.** Master's thesis, Informatics and Mathematical Modelling. Technical University of Denmark, 2000
- **S. BAKER ; D. SCHARSTEIN ; J. LEWIS ; S. ROTH ; M. J. BLACK ; R. SZELISKI.** A database and evaluation methodology for optical flow. *ICCV,* 2007
- **WEDEL, T. POCK ; D. CREMERS.** Structure- and motion-adaptive regularization for high accuracy optic flow. *ICCV,* 2009
- **WEDEL, T. POCK ; C. ZACH ; D. CREMERS ; H. BISCHOF.** An improved algorithm for TV-L1 optical flow. *In Dagstuhl Motion Workshop,* 2008
- **M. WERLBERGER ; W. TROBIN ; T. POCK ; A. WEDEL ; D. CREMERS ; H. BISCHOF.** Anisotropic Huber-L1 optical flow. *In BMVC,* 2009
- **H. ZIMMER ; A. BRUHN ; J. WEICKERT ; L. VALGAERTS ; A. SALGADO ; B. ROSENHAHN ; H.-P. SEIDEL.** Complementary optic flow. *In EM-MCVPR,* 2009
- Automatic face analysis in static and dynamic environments. **M. SORCI.** PhD. Thesis, EPFL. 2009
- **MALLAMPATI SR ; GATT SP ; GUGINO LD ; WARAKSA B ; FREIBURGER D ; LIU PL.** A Clinical sign to predict difficult intubation; A prospective study. *Can Anaesth Soc J,* 1985, vol. 32, 429-434
- **SAMSOON GLT ; YOUNG JRB.** Difficult tracheal intubation : a retrospective study. *Anaesthesia,* 1987, vol. 42, 487-490